# EUROPEAN PATENT SPECIFICATION

(11) **EP 1 192 944 B1**
(45) Date of publication and mention of the grant of the patent: **01.12.2004**
(21) Application number: 01121804.7
(22) Date of filing: 11.09.2001
(51) Int. Cl.: A61K 31/197, A61P 11/06, A61P 11/08

(54) **Use of pregabalin for treating asthma**
Verwendung von Pregabalin zur Asthma Behandlung
Utilisation de pregabaline dans le traitement de l'asthme

(30) Priority: 22.09.2000 US 234827 P
(43) Date of publication of application: 03.04.2002
(73) Proprietor: Warner-Lambert Company LLC, Morris Plains, New Jersey 07950 (US)
(72) Inventor: Schrier, Denis J., Ann Arbor, Michigan 48105 (US); Taylor, Charles Price Jr., Ann Arbor, Michigan 48105 (US)
(74) Representative: Tesch, Rudolf, Dr.

(56) References cited:
- WO-A-00/67742
- WO-A-92/09560

## Description

### FIELD OF THE INVENTION

This invention relates to the use of pregabalin for the manufacture of pharmaceutical compositions for preventing asthma and related inflammatory disease in mammals. This invention also relates to a method for treating or alleviating the symptoms of asthma and related inflammatory disease in mammals.

### BACKGROUND OF THE INVENTION

Asthma is a lung disease characterized by (1) airways obstruction that is reversible (but not completely in some patients), either spontaneously or with treatments, (2) airways inflammation, and (3) increased airways responsiveness to a variety of stimuli. Asthma involves airways obstruction which is due to a combination of factors that include (1) spasm of airways smooth muscle; (2) edema of airways mucosa; (3) increased mucus secretion; (4) cellular, especially eosinophilic, infiltration of the airways walls; and (5) injury and desquamation of the airways epithelium (see generally The Merck Manual of Diagnosis and Therapy, 7th edition, Berkow R. ed., Merck Research Laboratories, Rahway, NJ, 1992:34). About 10 million asthmatics live in the United States. From 1980 to 1987 there has been a 29% increase in the prevalence rates of asthma, and from 1970 to 1987 hospital discharge rates for asthma nearly tripled. More alarmingly, the mortality rate from asthma worldwide is increasing, in the United States alone increasing 37% from 1980 to 1987.

The alleviation or prevention of asthma related inflammation is highly desirable, for both human reasons and as an adjunct to proper effective clinical management of asthmatic disease. Aside from prophylactic steps to minimize exposure to certain environmental factors, and nonspecific exacerbating factors, treatment of asthma is conveniently considered as management of the acute attack and day-to-day therapy. Conventional methods of treatment can be classified under five groups of drug therapies: beta-adrenergic agents, theophylline, corticosteroids, cromolyn sodium, and anticholinergic agents (Windt M., "Asthma: Evolving Therapeutic Regimens," SPECTRUM Phamaceuticals, Decision Resources, Inc., 1991:23-1 to -7).

The pathophysiology of asthma is marked by an inflammatory state in which the airways are narrowed chronically by edema and episodically by a variety of spasmogens that are released from resident and infiltrating cells (see generally Goodman and Gilman's, The Pharmacological Basis of Therapeutics, 8th edition, Gilman, Rall, Nies, and Taylor eds., Pergamon Press, New York, NY, 1990;25). Asthmatic hyperresponsiveness to chemical and physical stimuli can cause bronchoconstriction almost immediately (immediate response), as well as an episode several hours later (late response). This late response appears to occur due to the recruitment of circulating cells which are attracted to the area by chemotactic factors (mediators), which serve to maintain or intensify the inflammatory state. It is believed that environmental antigens are responsible for maintaining a state of hyperresponsiveness which underlies the asthma of many individuals. The list of known or suspected mediators which may play a role in the allergic responses of asthma have been growing steadily over the years and currently include histamine, prostanoids, leukotrienes, and platelet-activating factor (PAF). Also implicated are the release of acetylcholine and neuropeptides such as substance P from parasympathetic nerves.

The study of the pathophysiology has been hampered by the lack of affordable, uniform, and well-characterized animal models for the study of atopic asthma. Atopic asthma in humans has been described as an allergic disease characterized by reversible obstruction of the airways or bronchi. The immune response associated with the onset of asthma has been described as having mixed histopathological features of both acute and chronic, cell-mediated immune reactions. This response is histopathologically characterized by the infiltration of the bronchial mucosa with neutrophils, eosinophils, macrophages, and lymphocytes (see for review Corrigan C.J. and Kay A.B., "T cells and eosinophils in the pathogenesis of asthma," *Immunol ToDay*, 1992; 13:501-507), though basophils have been indicated as being a source of chemotactic factors. Data on asthma suggests that the onset of the asthmatic response is controlled by CD4 + T-lymphocytes which produce a characteristic TH2 pattern of lymphokine production (Kay A.B. and Corrigan C.J., "Asthma, eosinophils and neutrophils," *Br Med Bull,* 1992;48:51-64). The TH2 pattern of lymphokines consists of expression of IL-4, IL-5, and IL-10 (Mossman and Moore, "The role of IL-10 in crossregulation of Th1 and Th2 response," *Immunol ToDay*, 1989;12:A49-A58; Mossmann T.R. et al., "Two types of murine helper T cell clone," *J Immunol,* 1986;136:2348-2359). The expression of these lymphokines correlates well with asthma as their individual functions play a role in the asthmatic response. The expression of IgE (IL-4) (Zhang X. et al., "T cells from atopic individuals produce IgE-inducing activity incompletely blocked by anti-interleukin-4 antibody," *Eur J Immunol,* 1992;22:829-833) and eosinophilia (IL-4/IL-5) (Spry C.J. et al., "Eosinophils," *Immunol ToDay*, 1992;13:384-387) are both characteristic of asthmatic responses (Del Prete G., "Human Th1 and Th2 lymphocytes: their role in the pathophysiology of atopy," *Allergy,* 1992;47:450-455). However, a primary trait of asthma is the accumulation of eosinophils in the bronchoalveolar lavage (BAL) fluid (Corrigan and Kay, 1992; Kay and Corrigan, 1992; Arm J.P. and Lee T.H., "The pathobiology of bronchial asthma," *Adv Immunol,* 1992;51:323-382; Diaz et al., "Leukocytes and mediators in bronchoalveolar lavage during allergen-induced late-phase asthmatic reactions." *Am Rev Respir Dis*, 1989;139:1383-1389). Historically, eosinophils have been implicated as a primary cell responsible for the induction of bronchial mucosal injury and are thought to induce the bronchial obstruction associated with the asthmatic response (Kay and Corrigan, 1992; Djukanovic et al., "Mucosal inflammation in asthma," *Am J Respir Dis*, 1990;142:434-457; Walker et al., "Increased expression of CD11b and functional changes in eosinophils after migration across endothelial cell monolayers," *J lmmunol,* 1993;150:4061-4071). Due to the difficulty of longitudinal studies of patient populations and paucity of asthma-related data in animal models, the mechanisms which lead to the pathophysiology of this disease are not presently clear. It would be most useful to the medical and scientific community to have a murine model to examine the cellular and molecular events involved in the asthmatic response.

In order to develop and test pharmaceutical treatments for asthma, it would be useful to have an appropriate, characterizable, economical, and readily available animal model system for the disease. Laboratories have employed, as animal models, the use of various antigens or pharmaceutical agents in dogs and primates as well as guinea pigs (Mapp et al., "Airway responsiveness to inhaled antigen, histamine, and methacholine in inbred, ragweed sensitive dogs," *Am Rev Respir Dis,* 1985;132:292-298; Sasaki et al., "Late asthmatic response to Ascaris antigen challenge in dogs treated with mtyrapone," *Am Rev Respir Dis*, 1989;136:1459-1465; Yamada et al., "Development of an animal model of late asthmatic response in guinea pigs and effects of anti-asthmatic drugs," *Prostaglandins,* 1992;43:507-521). Work with the canine system has not been greatly expanded and is hampered by the cost of the animals, care, lack of characterized reagents, ease of generating large numbers of genetically identical individuals, and strict regulations.

At present two animal models are mainly being used to study asthma. The first is an Ascaris suum parasite antigen-induced primate model system (Gundel R.H. et al., "Antigen-induced acute and late-phase responses in primates," *Am Rev Respir Dis*, 1992;146:369-373; Pritchard D.I. et al., "Laboratory infection of primates with Ascaris suum to provide a model of allergic bronchoconstriction," *Clin Exp Immunol,* 1983;54:469-476).

The second animal model being used is induced in guinea pigs by various antigens (Iijima H. et al., "Bronchoalveolar lavage and histologic characterization of late asthmatic response in guinea pigs," *Am Rev Respir Dis,* 1987;136:922-929; Ishida K. et al, "Repeated antigen challenge induces airway hyperresponsiveness with tissue eosinophilia in guinea pigs," *J Appl Physiol,* 1989;67:1133-1139; Vertes C. et al., "A model for experimental asthma: provocation in guinea-pigs immunized with Bordetella perussis," *Bull Eur Physiopathol Respir,* 1987;10:111s-113s), and pharmaceuticals (Hayes J.P. et al., "Bronchoconstriction and airway microvascular leakage in guinea pigs sensitized with trimellitic anhydride," *Am Rev Respir Dis,* 1992;146:1306-1310; Obata H. et al., "Guinea pig model of immunologic asthma induced by inhalation of trimellitic anhydride," *Am Rev Respir Dis*, 1992;146:1553-1558).

Generally, the focus of therapeutic intervention of the mechanisms of asthma has focused on inhibition or antagonism of chemical mediators involved in cell recruitment, potentiation, and maintenance of the hyperresponsive state. Such therapies aim to reduce the activation or accumulation of inflammatory cells in the sensitive regions (Morley J., "Immunopharmacology of asthma," TiPS, 1993;14:208-213). As appreciation of the complexity of the pathophysiology of asthma has increased, certainty as to the mechanism of action and the cellular targets of therapeutic agents has decreased. This is a major limitation to the use of mediator based pharmaceuticals for treating asthma, which is in addition to the unwanted side effects associated with such treatments. Atopy has become a focus for some researchers, and directs pharmacological intervention of mediators and cells on the immunological/immunogenic basis of asthma. (Djkanovic R. et al., "Mechanisms of airways inflammation which may be amenable to prophylaxis," *AAS Update on Childhood Asthma*, 1993;40:169-180). Many cytokines have been studied with the goal of determining the cell sources (Ackerman V. et al., "Detection of cytokines and their cell sources in bronchial biopsy specimens from asthmatic patients," *Chest,* 1994;105:687-696). The predominant cells bearing IgE in the late-phase response to antigen in the lung was identified as being basophils in one study focusing on histamine as a key mediator. (Guo C.B. et al., "Identification of IgE-bearing cells in the late-phase response to antigen in the lung as basophils," *Am J Respir Cell Mol Biol,* 1994;10:384-390). Thus, while the focus is still on reduction of hyperreactivity, and reducing inflammation by intervention with the mediator signals, it has been recognized that a reduction in the number of reactive cells will apparently result in reduced inflammation (Godfrey S., "Airway inflammation, bronchial reactivity and asthma," *AAS Update on Childhood Asthma*, 1993;40:109-143).

It would, therefore, be advantageous to be able to utilize a pharmaceutical composition and a treatment which produce minimal toxicity or side effects, but provide highly effective, reproducible results yielding a reduction in the number of reactive cells, thereby avoiding the complexity and confusion over therapeutic intervention with mediator signals, by preventing the action of the responsible cells. There remains a need in the medical arts for an effective treatment for asthma, and the inflammation that is associated with asthma.

### SUMMARY OF THE INVENTION

The present invention relates to the use of an amount of Formula I which is active in bronchial tissue and is sufficient to reduce bronchial spasms associated with asthma for the manufacture of a medicament for preventing bronchial disorders such as asthma, and related inflammatory disorders in an individual 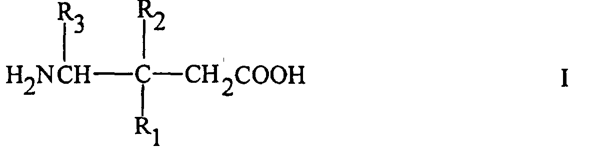 wherein R₁ is a straight or branched alkyl of from 1 to 6 carbon atoms, phenyl, or cycloalkyl having from 3 to 6 carbon atoms; R₂ is hydrogen or methyl; and R₃ is hydrogen, methyl, or carboxyl; or an individual diastereomeric or enantiomeric isomer thereof; or a pharmaceutically acceptable salt, ester, amide or prodrug thereof. The most preferred compound of Formula I is where R₂ and R₃ are both hydrogen, and R₁ is -(CH₂)₀₋₂-iC₄H₉ as an ( R), (S), or (R,S) isomer. A more preferred embodiment of the invention utilizes 3-aminomethyl-5-methyl-hexanoic acid, and especially (S)-3-(aminomethyl)-5-methylhexanoic acid, now known generically as pregabalin. Another preferred compound is 3-(1-aminoethyl)-5-methylhexanoic acid.

The present invention is also directed to the use of an amount of Formula I, for the manufacture of a medicament for treating on alleviating the symptoms of bronchial disorders, such as asthma, and related inflammatory disease in an individual.

### DETAILED DESCRIPTION OF THE INVENTION

The instant invention is the use of a compound of Formula I above as an antiasthmatic in the treatment of asthma and related inflammatory conditions as listed above. The condition of asthma is well understood to those skilled in the art.

The terms used in Formula I are, for example, alkyl which term is methyl, ethyl, propyl, isopropyl, n-butyl, isobutyl, sec-butyl, tert-butyl, isopentyl, and neopentyl, as well as those as would occur to one skilled in the art.

The term "cycloalkyl" is exemplified by cyclopropyl, cyclobutyl, cyclopentyl, and cyclohexyl.

The compounds of the Formula I are known to those skilled in the art of organic chemistry and the synthetic methodology is set forth in U.S. Patent No. 5,563,175, where the compounds are described as anti-seizure agents.

The compounds of the present invention may form pharmaceutically acceptable salts with both organic and inorganic acids or bases. For example, the acid addition salts of the basic compounds are prepared either by dissolving the free base in aqueous or aqueous alcohol solution or other suitable solvents containing the appropriate acid and isolating the salt by evaporating the solution. Examples of pharmaceutically acceptable salts are hydrochlorides, hydrobromides, hydrosulfates, etc. as well as sodium, potassium, and magnesium, etc. salts.

The compounds of the present invention can contain one or several asymmetric carbon atoms. The invention includes the individual diastereomers or enantiomers, and the mixtures thereof. The individual diastereomers or enantiomers may be prepared or isolated by methods already well-known in the art.

The compounds made by the synthetic methods can be used as pharmaceutical compositions as agent in the prevention/treatment or alleviation of symptoms of asthma and related inflammatory disorders when an effective amount of a compound of the Formula I, together with a pharmaceutically acceptable carrier is used. The pharmaceutical can be used in a method for treating such disorders in mammals, including human, suffering therefrom by administering to such mammals an effective amount of the compound as described above in unit dosage form.

The present invention relies on the bronchodilation activity of the compounds of Formula I to provide relief from bronchial disorders. In the present method, one or more compounds of Formula I, is administered alone, or in combination with one or more other drug(s) in adjunctive treatment, to an individual in whom asthma relief (e.g., relief from bronchial spasms, shortness of breath) is desired. In the present method, a compound of Formula I is administered to an individual who has asthma. For example, pregabalin is administered to an individual after onset of asthma to reduce breathing difficulty resulting from asthma. In another embodiment, pregabalin is administered prophylactically, that is, before the bronchiospasm begins in an asthma attack, to prevent its occurrence or to reduce the extent to which it occurs.

In the present use, a compound of Formula I can be administered by inhalation, by subcutaneous, intramuscular or other injection, orally, intravenously, topically, parenterally, transdermally, rectally or via an implanted reservoir containing the drug. The form in which the drug will be administered (e.g., inhalant, powder, tablet, capsule, solution, emulsion) will depend on the route by which it is administered. The drug also can be administered in sustained-release dosage forms, either orally or topically, e.g., by transdermal delivery from a topically applied patch or similar device. The quantity of the drug to be administered will be determined on an individual basis, and will be based at least in part on consideration of the individual's size, the severity of the symptoms to be treated and the result sought. In general, quantities of a compound of Formula I sufficient to reduce the symptoms of asthma will be administered. The actual dosage (quantity administered at a time) and the number of administrations per day will depend on the mode of administration, for example, by inhaler, nebulizer, topical or oral administration. About 25 mcg to about 50 mcg of a compound of Formula I given by inhalation one or more times per day will be adequate in most individuals to produce the desired bronchodilation effect. For oral administration, e.g., tablet or syrup, a dose of about 1 mg to about 8 mg two to four times daily is administered to produce the desired effect.

In the use of the present invention, a compound of Formula I can be administered together with one or more other drug(s). For example, an antiasthmatic drug such as theophylline or terbutaline, or antihistamine or analgesic such as aspirin, acetaminophen or ibuprofen, can be given with or in close temporal proximity to administration of a compound of Formula I. The two (or more) drugs (i.e., the compound of Formula I and another drug) can be administered in one composition or as two separate entities. For example, they can be administered in a single capsule, tablet, powder, or liquid, etc. or as individual compounds. The components included in a particular composition, in addition to a compound of Formula I and another drug or drugs, are determined primarily by the manner in which the composition is to be administered. For example, a composition to be administered in inhalant form can include, in addition to the drug(s), a liquid carrier and/or propellent. A composition to be administered in tablet form can include a filler (e.g., lactose), a binder (eg, carboxymethyl cellulose, gum arabic, gelatin), an adjuvant, a flavoring agent, a coloring agent and a coating material (e.g., wax or a plasticizer). A composition to be administered in liquid form can include the combination of drugs and, optionally, an emulsifying agent, a flavoring agent and/or a coloring agent.

In general, according to the use of the present invention, a compound of Formula I, alone or in combination with another drug(s), is administered to an individual periodically as necessary to reduce symptoms of asthma.

### EXAMPLES

The invention is further defined by reference to the following procedures describing the pharmacological characterization of the compositions of the present invention.

| Tablet Formulation | |
|---|---|
| Pregabalin | 300 mg |
| Magnesium stearate | 20 mg |
| Microcrystalline cellulose | 100 mg |
| Povidone | 100 mg |
| Talc | 50 mg |

The ingredients are blended to uniformity and pressed into a tablet. The tablets are administered from 1 to 3 times a day for treatment of asthmatic conditions.

| Capsule Formulation | |
|---|---|
| Pregabalin | 50 mg |
| Cellulose | 100 mg |
| Gelatin | 50 mg |
| Titanium dioxide | 10 mg |
| Cornstarch | 50 mg |

The ingredients are blended to uniformity and placed into a gelatin capsule. The capsules are administered from 1 to 4 times a day for treatment of asthmatic conditions.

## Claims

1. Use of a compound of general formula I: 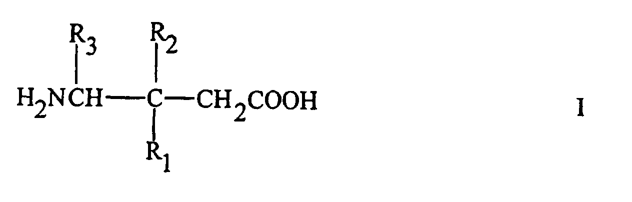 wherein
R₁ is a straight or branched alkyl of from 1 to 6 carbon atoms, phenyl, or cycloalkyl having from 3 to 6 carbon atoms;
R₂ is hydrogen or methyl; and
R₃ is hydrogen, methyl, or carboxyl; or an individual diastereomeric or enantiomeric isomer thereof; or a pharmaceutically acceptable salt, ester, amide or prodrug thereof for the manufacturing of pharmaceutical compositions for treating or alleviating the symptoms of asthma or related inflammatory disorders by bronchodilation.

2. Use of a compound of general formula I according to claim 1 for the manufacturing of pharmaceutical compositions for preventing asthma in an individual by preventing bronchoconstriction.

3. The use of claim 1, wherein the compound is pregabalin.

4. The use of claim 2, wherein the compound is pregabalin.

5. The use of claim 1, wherein the pharmaceutical composition is designed for the inhalation of 25 µg to 50 µg of the compound by an individual.

6. The use of claim 2, wherein the pharmaceutical composition is designed for the inhalation of 25 µg to 50 µg of the compound to an individual.

7. The use of claim 1, wherein the pharmaceutical composition is designed for the oral administration of 1 mg to 8 mg g to an individual 1 to 4 times daily.

8. The use of claim 2, wherein the pharmaceutical composition is designed for the oral administration of 1 mg to 8 mg g to an individual 1 to 4 times daily.

9. The use of claim 1, wherein the pharmaceutical composition is designed to result in sufficient bronchodilation and to be administered with at least one additional drug.

10. The use of claim 1, wherein the pharmaceutical composition is designed to prevent sufficiently bronchconstriction and to be administered with at least one additional drug.

## Patentansprüche

1. Die Verwendung einer Verbindung der allgemeinen Formel I: 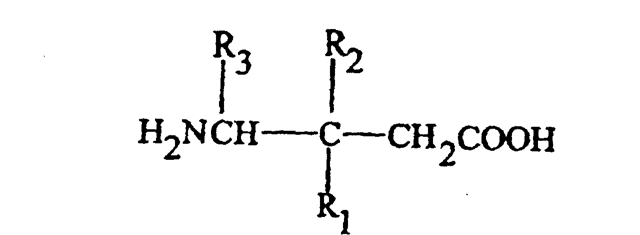 worin
R₁ eine unverzweigte oder verzweigte Alkylgruppe bestehend aus 1 bis 6 Kohlenstoffatomen, eine Phenylgruppe oder eine cylische Alkylgruppe mit 3 bis 6 Kohlenstoffatomen ist;
R₂ ein Wasserstoffatom oder eine Methylgruppe ist, und
R3 ein Wasserstoffatom, eine Methylgruppe oder eine Carboxylgruppe ist; oder ein individuelles Diastereomer oder ein enantiomeres Isomer hiervon; oder ein pharmazeutisch akzeptables Salz, Ester, Amid oder Pro-Pharmakon (Prodrug) hiervon zur Herstellung pharmazeutischer Zubereitungen zur Behandlung oder Linderung der Symptome von Asthma oder verwandten entzündlichen Erkrankungen durch Dilatation der Bronchien.

2. Die Verwendung einer Verbindung der allgemeinen Formel I gemäß Anspruch 1 zur Herstellung pharmazeutischer Zubereitungen zur Asthmaprävention an Individuen durch Verhinderung der Konstriktion der Bronchien.

3. Die Verwendung von Anspruch 1, worin die Verbindung Pregabalin ist.

4. Die Verwendung von Anspruch 2, worin die Verbindung Pregabalin ist.

5. Die Verwendung von Anspruch 1, worin die pharmazeutische Zubereitung für die Inhalation von 25µg bis 50µg der Verbindung bei einem Individuum konzipiert ist.

6. Die Verwendung von Anspruch 2, worin die pharmazeutische Zubereitung für die Inhalation von 25µg bis 50µg der Verbindung bei einem Individuum konzipiert ist.

7. Die Verwendung von Anspruch 1, worin die pharmazeutische Zubereitung für die orale Anwendung von 1 mg bis 8 mg der Verbindung bei einem Individuum ein bis viermal täglich konzipiert ist.

8. Die Verwendung von Anspruch 2, worin die pharmazeutische Zubereitung für die orale Anwendung der Verbindung bei einem Individuum von 1 mg bis 8 mg ein bis viermal täglich konzipiert ist.

9. Die Verwendung von Anspruch 1, worin die pharmazeutische Zubereitung dergestalt konzipiert ist, dass sie eine ausreichende Dilatation der Bronchien bewirkt und dass sie mit zumindest einem weiteren Wirkstoff verabreicht werden kann.

10. Die Verwendung von Anspruch 1, worin die pharmazeutische Zubereitung dergestalt konzipiert ist, dass diese die Konstriktion der Bronchien genügend wirksam verhindert und dass sie mit zumindest einem weiteren Wirkstoff verabreicht werden kann.

## Revendications

1. Utilisation d'un composé répondant à la formule générale I: 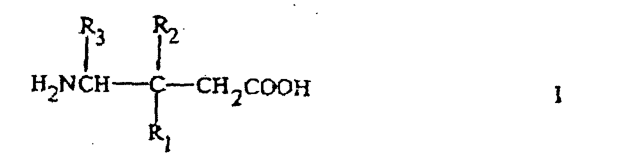 dans laquelle
R₁ est un alkyle linéaire ou ramifié de 1 à 6 atomes de carbone, phényle ou cycloalkyle ayant de 3 à 6 atomes de carbone;
R₂ est hydrogène ou méthyle; et
R₃ est hydrogène, méthyle ou carboxyle; ou un isomère individuel de ceux-ci, diastéréoisomère ou énantiomère ; ou un sel, ester, amide ou pro-médicament pharmaceutiquement acceptable de ceux-ci, pour la fabrication de compositions pharmaceutiques destinées à traiter ou à atténuer les symptômes de l'asthme ou des troubles inflammatoires apparentés, au moyen de la bronchodilatation.

2. Utilisation d'un composé de formule générale I selon la revendication 1 pour la fabrication de compositions pharmaceutiques destinées à prévenir l'asthme chez un individu en prévenant la bronchoconstriction.

3. Utilisation de la revendication 1, dans laquelle le composé est la prégabaline.

4. Utilisation de la revendication 2, dans laquelle le composé est la prégabaline.

5. Utilisation de la revendication 1, dans laquelle la composition pharmaceutique est conçue pour l'inhalation de 25 µg à 50 µg du composé par un individu.

6. Utilisation de la revendication 2, dans laquelle la composition pharmaceutique est conçue pour l'inhalation de 25 µg à 50 µg du composé par un individu.

7. Utilisation de la revendication 1, dans laquelle la composition pharmaceutique est conçue pour l'administration orale de 1 mg à 8 mg à un individu 1 à 4 fois par jour.

8. Utilisation de la revendication 2, dans laquelle la composition pharmaceutique est conçue pour l'administration orale de 1 mg à 8 mg à un individu 1 à 4 fois par jour.

9. Utilisation de la revendication 1, dans laquelle la composition pharmaceutique est conçue de manière à donner lieu à une bronchodilatation suffisante et pour être administrée en concomitance avec au moins un médicament supplémentaire.

10. Utilisation de la revendication 1, dans laquelle la composition pharmaceutique est conçue de manière à prévenir suffisamment la bronchoconstriction et pour être administrée en concomitance avec au moins un médicament supplémentaire.
